# EUROPEAN PATENT SPECIFICATION

(11) **EP 4 327 898 B1**
(45) Date of publication and mention of the grant of the patent: **22.10.2025**
(21) Application number: 21942952.9
(22) Date of filing: 25.05.2021
(51) Int. Cl.: A63B 71/06, A61B 5/00

(54) **RUNNING STYLE ANALYSIS DEVICE, RUNNING STYLE ANALYSIS METHOD, AND RUNNING STYLE ANALYSIS PROGRAM**
VORRICHTUNG ZUR LAUFSTILANALYSE, VERFAHREN ZUR LAUFSTILANALYSE UND PROGRAMM ZUR LAUFSTILANALYSE
DISPOSITIF D'ANALYSE DE STYLE DE COURSE, PROCÉDÉ D'ANALYSE DE STYLE DE COURSE, ET PROGRAMME D'ANALYSE DE STYLE DE COURSE

(43) Date of publication of application: 28.02.2024
(73) Proprietor: ASICS Corporation, Kobe-shi, Hyogo 650-8555 (JP)
(72) Inventor: SAKAGUCHI, Masanori, Kobe-shi, Hyogo 650-8555 (JP); NAKAYA, Seigo, Kobe-shi, Hyogo 650-8555 (JP); HIRAKAWA, Nao, Kobe-shi, Hyogo 650-8555 (JP); TANIGUCHI, Norihiko, Kobe-shi, Hyogo 650-8555 (JP)
(74) Representative: Marks & Clerk LLP
(86) International application number: PCT/JP2021/019866
(87) International publication number: WO 2022/249298

(56) References cited:
- EP-A1- 3 199 046
- EP-A1- 3 626 169
- JP-A- 2001 170 029
- JP-A- 2016 214 499
- JP-A- 2017 029 383
- JP-A- 2019 063 482
- JP-A- 2019 084 113
- JP-B2- 4 856 427
- US-A1- 2011 184 225
- US-A1- 2014 156 215
- "Correlation graph of strides and running paces", NOTE BLOG, 15 January 2012 (2012-01-15), pages 1 - 8, Retrieved from the Internet <URL:https://87889070_at.webry.info/201501/article_10.html> [retrieved on 20190422]

## Description

### 1. TECHNICAL FIELD

The present invention relates to a technology for analyzing running styles of marathon runners.

### 2. DESCRIPTION OF THE RELATEDART

In long-distance running such as marathons, "high cadence running" and "long stride running" are known as running styles of runners. In general, the former is considered as a running style with relatively high cadence (the number of steps per unit time) and relatively short stride length (the length of one step), and the latter as a running style with relatively low cadence and relatively long stride length; however, there is no clear definition.

In recent years, running shoes suitable for the high cadence running and those suitable for the long stride running have been developed respectively. Therefore, a runner may be able to select more suitable shoes by knowing whether his or her running style is the high cadence type or the long stride type (see Patent Literature 1, for example).

Patent literature 2 shows a prior art running style analysis device.

### PATENT LITERATURE

Patent Literature 1: Japanese Patent No. 4856427
Patent literature 2: US 2014/156215 A1

### SUMMARY

However, except in cases where the tendency of the cadence or the stride length is particularly pronounced, there have been conventionally no clear criteria for determining whether the running style of a runner falls under the high cadence type or the long stride type. Accordingly, such determining has had to rely on subjective judgment.

Under such circumstances, as a result of analyzing the running records of a large number of runners, the inventors have found a method for distinguishing between the both types, with objective criteria based on the running style tendency.

The present invention has been made in view of such an issue, and a purpose thereof is to provide a technology for analyzing the running style of a runner.

In response to the above issue, a running style analysis device according to one embodiment of the present invention includes: a first cadence acquirer that acquires, with regard to running of a subject, a cadence at a first running speed as a first cadence; a second cadence acquirer that acquires, with regard to running of the subject, a cadence at a second running speed that is different from the first running speed, as a second cadence; a judgment unit that makes judgment as to which of multiple running style types, which include a long stride type and a high cadence type, running of the subject falls under, based on the difference between the first cadence and the second cadence with respect to the difference between the first running speed and the second running speed; and a result output unit that outputs a result of the judgment.

The "running style analysis device" may be implemented by a combination of a server and a server program running on a web server or in the cloud, or by a combination of a device, such as a smartphone, a tablet, any other information terminal, or a personal computer, and a program running on such a device. Alternatively, the running style analysis device may be implemented by a combination of a wearable device with various sensors embedded therein, and a program running on such a wearable device. The "first cadence acquirer" and the "second cadence acquirer" may acquire cadence information from the running data of the subject measured in advance or may acquire cadence information from information regarding the subject during running acquired by a predetermined sensor. The "first running speed" and the "second running speed" may be set to relatively high speeds, mainly assuming advanced runners whose marathon completion time is within 3 hours. For example, two running speeds with a difference of 1 m/s therebetween included in the range of 4.17 m/s (corresponding to 4 min/km pace) to 5.56 m/s (corresponding to 3 min/km pace) may be set as the first running speed and the second running speed. The increase in stride length with respect to the increase in running speed may tend to be greater in the long stride type than in the high cadence type, and the increase in cadence with respect to the increase in running speed may tend to be greater in the high cadence type than in the long stride type. The "judgment unit" may judge the running style type based on the increase in cadence with respect to the increase in running speed in the subject's running. The "multiple running style types" include the long stride type and the high cadence type and may further include an intermediate type positioned therebetween. According to this embodiment, whether the runner's running style is the high cadence type or the long stride type can be judged simply by acquiring the cadence at multiple running speeds and analyzing the cadence change with respect to the speed change, thereby obtaining information useful for shoe selection.

When at least one of the first cadence or the second cadence is greater than or equal to a judgment criterion value obtained based on a predetermined criterion, the judgment unit may judge that the subject's running falls under the high cadence type, regardless of the difference in cadence. The "judgment criterion value obtained based on a predetermined criterion" may be a variable value calculated using a certain linear equation with the running speed as a variable or may be a certain fixed value determined in advance based on an experiment, analysis, or knowledge. According to this embodiment, when the runner's cadence is greater than or equal to the judgment criterion value, the running can be immediately judged as the high cadence type without any other calculation or analysis, simplifying the judgment method.

The judgment unit may judge that the subject's running falls under the high cadence type when the difference in cadence is within a predetermined first reference range and may judge that the subject's running falls under the long stride type when the difference in cadence is within a predetermined second reference range, which is lower than the first reference range. The "difference in cadence" may be the increase in cadence with respect to the increase in running speed in the subject's running. The entire range of cadence may be divided into two ranges of the "first reference range" and the "second reference range" with a predetermined value as the boundary, so that which one the difference in cadence is included in may be judged. Also, the entire range of cadence may be divided into multiple reference ranges including other reference ranges, so that which one the difference in cadence is included in may be judged. According to this embodiment, as long as the difference in cadence between multiple running speeds in the subject's running can be acquired, whether the running falls under the high cadence type or the long stride type can be accurately judged.

The judgment unit may judge that the subject's running falls under the high cadence type when the difference in cadence is within a predetermined first reference range, may judge that the subject's running falls under the long stride type when the difference in cadence is within a predetermined second reference range, which is lower than the first reference range, and may judge that the subject's running falls under an intermediate type when the difference in cadence is within a predetermined third reference range, which is lower than the first reference range and higher than the second reference range. The "intermediate type" may refer to a running style that cannot be said to be either the high cadence type or the long stride type, or a running style that does not have remarkable characteristics but can be said to fall under either the high cadence type or the long stride type, if anything. According to this embodiment, as long as the difference in cadence between multiple running speeds in the subject's running can be acquired, whether the running falls under the high cadence type, the long stride type, or the intermediate type can be accurately judged.

The running style analysis device may further include a recommendation output unit that outputs, based on a result of the judgment, information recommending at least one of multiple shoes including a shoe suitable for a high cadence type runner and a shoe suitable for a long stride type runner.

According to this embodiment, as long as the difference in cadence between multiple running speeds in the subject's running can be acquired, information on shoes suitable for the running style can be accurately obtained.

Another embodiment of the present invention relates to a running style analysis method. The method includes: acquiring, with regard to running of a subject, a cadence at a first running speed as a first cadence; acquiring, with regard to running of the subject, a cadence at a second running speed that is different from the first running speed, as a second cadence; making judgment as to which of multiple running style types, which include a long stride type and a high cadence type, the subject's running falls under, based on the difference between the first cadence and the second cadence with respect to the difference between the first running speed and the second running speed; and outputting a result of the judgment.

According to this embodiment, whether the runner's running style is the high cadence type or the long stride type can be judged simply by acquiring the cadence at multiple running speeds and analyzing the cadence change with respect to the speed change, thereby obtaining information useful for shoe selection.

Optional combinations of the aforementioned constituting elements, and implementation of the present invention, including the constituting elements and expressions, in the form of methods, apparatuses, programs, transitory or non-transitory storage medium storing programs, or systems may also be practiced as additional modes of the present invention.

### BRIEF DESCRIPTION OF DRAWINGS

[FIG. 1] FIG. 1 is a diagram that illustrates a basic configuration of a running style analysis system.
[FIGS. 2] FIGS. 2 are diagrams in which changes in stride length and changes in cadence are compared between the long stride type and the high cadence type in the same running speed range.
[FIGS. 3] FIGS. 3 are scatter plots that illustrate the relationships between the running speed and the stride length and the relationships between the running speed and the cadence of multiple long stride type runners.
[FIGS. 4] FIGS. 4 are scatter plots that illustrate the relationships between the running speed and the stride length and the relationships between the running speed and the cadence of multiple high cadence type runners.
[FIG. 5] FIG. 5 is a functional block diagram that shows a basic configuration of a user terminal.
[FIG. 6] FIG. 6 is a functional block diagram that shows a basic configuration of a running style analysis server.
[FIG. 7] FIG. 7 is a flowchart that shows basic processing performed in the running style analysis server.
[FIG. 8] FIG. 8 is a flowchart that shows the details of judgement processing at S20 in FIG. 7.
[FIG. 9] FIG. 9 is a diagram in which results of running style judgment based on the cadence are compared with experimental results, with regard to 14 subjects.

### DETAILED DESCRIPTION

In the following, the present invention will be described based on a preferred embodiment with reference to each drawing. In the embodiment and modifications, like reference characters denote like or corresponding constituting elements, and the repetitive description will be omitted as appropriate. In each drawing, parts less important in describing the embodiment may be omitted.

In the present embodiment, it is premised that a user, who is a runner and wants to know whether running shoes (hereinafter, simply referred to as "shoes") for high cadence running or shoes for long stride running are suitable for him or her, performs running style analysis by himself or herself. First, the user wears various wearable devices during running, acquires information necessary for analysis with various sensors, and transmits the information to the user's terminal. Thereafter, the user transmits the information from the terminal to a server and obtains analysis results from the server.

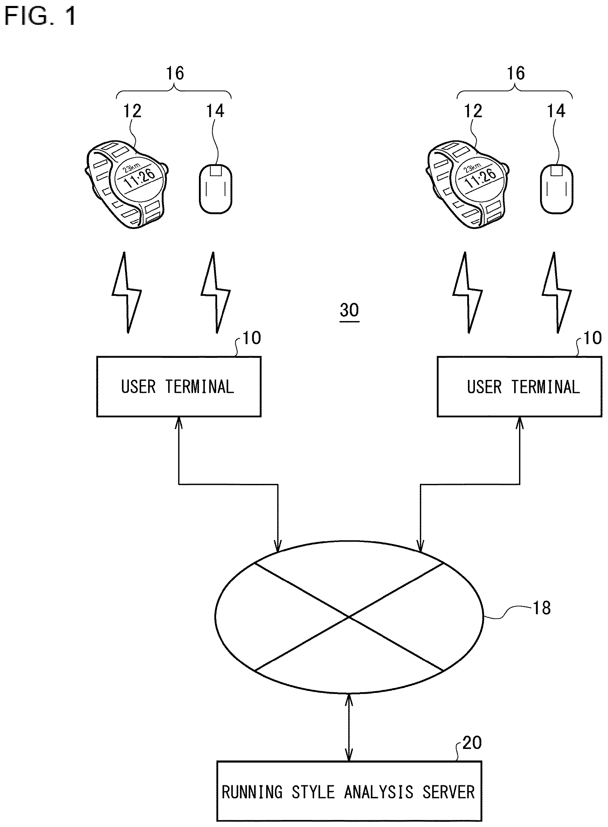

FIG. 1 illustrates a basic configuration of a running style analysis system. A running style analysis system 30 is constituted by a user terminal 10, a wearable device 16, and a running style analysis server 20, for example. A user performs running while wearing a wearable device 16, such as a running watch 12 and a motion sensor 14, on an arm or the waist and acquires various detection data with the running watch 12 and the motion sensor 14. The running watch 12 and the motion sensor 14 include sensors such as a positioning module and a 9-axis motion sensor. The running speed is acquired based on the relationship between time information and position information detected by the positioning module, and the cadence is acquired based on information detected by the 9-axis motion sensor. Also, the stride length is acquired based on the running distance measured by the positioning module and the cadence (stride length = running distance ÷ cadence). A running log acquired by the user terminal 10 is transmitted to the running style analysis server 20 via a network 18. Accordingly, the running style is analyzed by the running style analysis server 20, and which of multiple running styles, including the high cadence type and the long stride type, it corresponds to is judged.

In a modification, instead of a wearable device 16, a positioning module or a motion sensor built into a smartphone as the user terminal 10 may be used. In another modification, as data indicating the running state of the subject, the running speed data and cadence data may be acquired from images captured by a high-speed camera using a technology such as motion capture or may be acquired by detecting floor reaction force using a force plate. In such a case, an operator other than the user (e.g., a salesperson in a store) may operate the user terminal 10 to acquire the running state data for the subject and allow the running style analysis server 20 to perform running style analysis.

The "cadence" information is, for example, a numerical value in units of steps per minute (spm). When a runner whose marathon completion time is within 3 hours and 30 minutes runs at race pace, the cadence generally falls within the range of 175 to 205 spm on average. Also, the "stride length" (also called step length) information is an average step length (m), which is obtained by dividing the running distance per minute by the cadence.

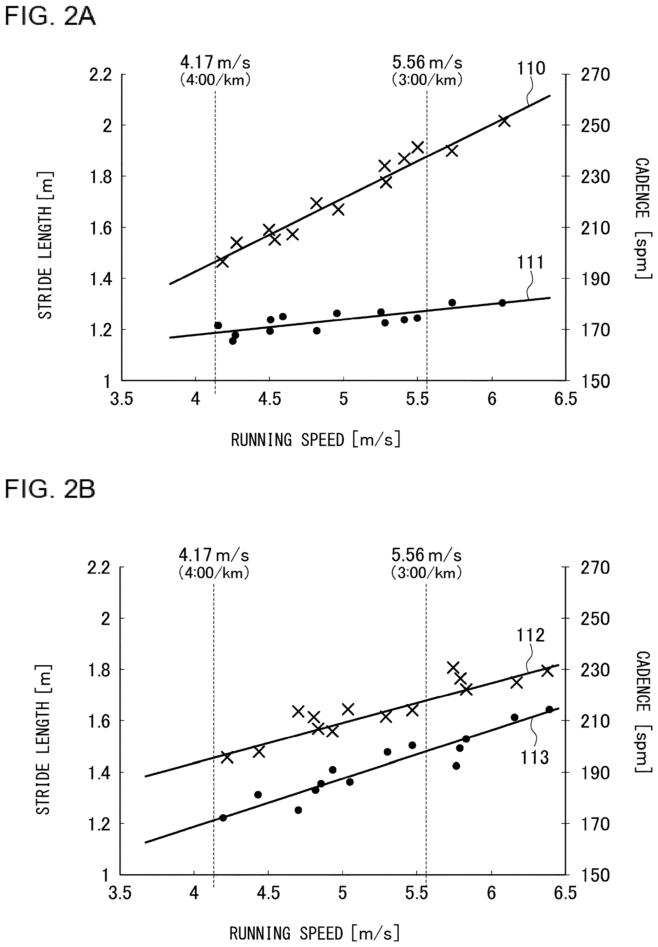

FIGS. 2 are diagrams in which changes in stride length and changes in cadence are compared between the long stride type and the high cadence type in the same running speed range. FIG. 2A is a scatter plot that illustrates the relationship between the running speed and the stride length and the relationship between the running speed and the cadence of a long stride type runner, whose personal best marathon completion time is 2 hours 36 minutes 7 seconds. FIG. 2B is a scatter plot that illustrates the relationship between the running speed and the stride length and the relationship between the running speed and the cadence of a high cadence type runner, whose personal best marathon completion time is 2 hours 40 minutes 0 seconds. The horizontal axis represents the running speed [m/s], and the vertical axis represents the stride length [m] or cadence [spm]. In FIGS. 2, the stride length ("×" marks) and the cadence ("·" marks) are plotted for the case of running in the range of 4.0 to 6.5 m/s, including 4.17 m/s (4 min/km pace) and about 5.56 m/s (3 min/km pace).

In the case of the long stride type runner shown in FIG. 2A, the stride length increases greatly by a wide range of about 0.6 m, from about 1.4 m to about 2 m, in proportion to the increase in running speed. The slope of a stride length change 110, which is a regression line indicating the increase in stride length with respect to the increase in running speed, is relatively large. In particular, with respect to the increase in running speed from 4.17 m/s (4 min/km pace) to about 5.56 m/s (3 min/km pace), the stride length increases by +0.39 m from 1.49 m to 1.88 m.

In contrast, the cadence of the long stride type runner gradually increases by a narrow range from 169 spm to 183 spm in proportion to the increase in running speed. The slope of a cadence change 111, which is a regression line indicating the increase in stride length with respect to the increase in running speed, is slight and nearly flat. In particular, with respect to the increase in running speed from 4.17 m/s (4 min/km pace) to about 5.56 m/s (3 min/km pace), the cadence increases by only +8 spm from 169 spm to 177 spm.

Meanwhile, in the case of the high cadence type runner shown in FIG. 2B, the stride length increases by a range of about 0.4 m, from about 1.4 m to about 1.8 m, in proportion to the increase in running speed. The slope of a stride length change 112, which is a regression line indicating the increase in stride length with respect to the increase in running speed, is smaller than that of the long stride type. In particular, with respect to the increase in running speed from 4.17 m/s (4 min/km pace) to about 5.56 m/s (3 min/km pace), the stride length increases by only +0.2 m from 1.48 m to 1.68 m.

In contrast, the cadence of the high cadence type runner increases greatly by a wide range from 170 spm to 230 spm in proportion to the increase in running speed. The slope of a cadence change 113, which is a regression line indicating the increase in cadence with respect to the increase in running speed, is greater than that of the long stride type. In particular, with respect to the increase in running speed from 4.17 m/s (4 min/km pace) to about 5.56 m/s (3 min/km pace), the cadence increases by +28 spm from 170 spm to 198 spm.

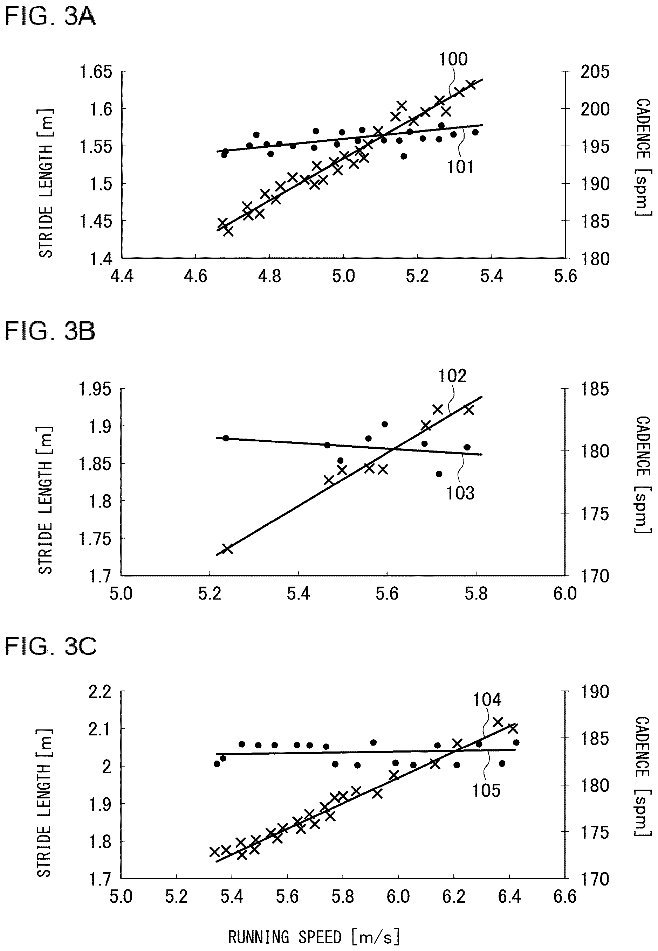

FIGS. 3 are scatter plots that illustrate the relationships between the running speed and the stride length and the relationships between the running speed and the cadence of multiple long stride type runners. The horizontal axis represents the running speed [m/s], and the vertical axis represents the stride length [m] or cadence [spm].

FIG. 3A shows an example in which a first runner ran in a range of about 4.67 m/s (about 3 minutes 34 seconds per kilometer pace) to about 5.37 m/s (about 3 minutes 6 seconds per kilometer pace). In this case, the stride length plotted with "×" marks increases greatly by a range of about 0.19 m, from about 1.44 m to about 1.63 m, in proportion to the increase in speed. The slope of a stride length change 100, which is a regression line indicating the change in stride length with respect to the change in running speed, is relatively large. In contrast, the cadence plotted with "·" marks only increases by a narrow range from 194 to 198 as the speed increases. A cadence change 101, which is a regression line indicating the change in pace with respect to the change in running speed, is almost flat or has a slight slope.

FIG. 3B shows an example in which a second runner ran in a range of about 5.23 m/s (about 3 minutes 11 seconds per kilometer pace) to about 5.78 m/s (about 2 minutes 53 seconds per kilometer pace). In this case, the stride length plotted with "×" marks increases greatly by a range of about 0.2 m, from about 1.73 m to about 1.93 m, in proportion to the increase in speed. The slope of a stride length change 102, which is a regression line indicating the increase in stride length with respect to the increase in running speed, is relatively large. In contrast, the cadence plotted with "·" marks only changes by a narrow range from 178 to 183 as the speed increases. A cadence change 103, which is a regression line indicating the change in pace with respect to the increase in running speed, is almost flat or even slightly negative.

FIG. 3C shows an example in which a third runner ran in a range of about 5.35 m/s (about 3 minutes 7 seconds per kilometer pace) to about 6.42 m/s (about 2 minutes 36 seconds per kilometer pace). In this case, the stride length plotted with "×" marks increases greatly by a range of about 0.26 m, from about 1.75 m to about 2.11 m, in proportion to the increase in speed. The slope of a stride length change 104, which is a regression line indicating the increase in stride length with respect to the increase in running speed, is relatively large. In contrast, the cadence plotted with "·" marks only changes by a narrow range from 182 to 185 as the speed increases. A cadence change 105, which is a regression line indicating the change in pace with respect to the increase in running speed, is almost flat.

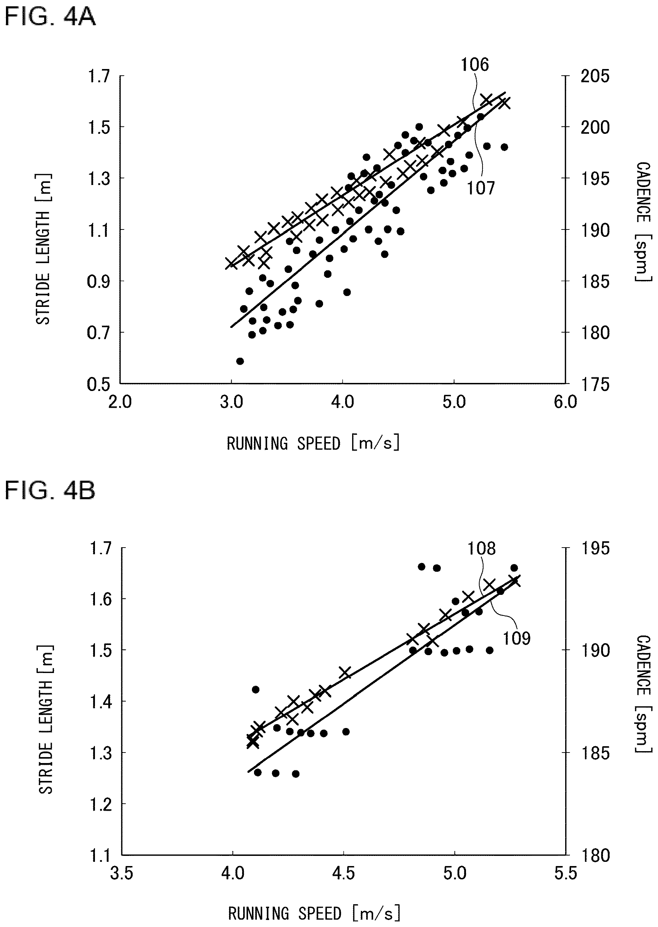

FIGS. 4 are scatter plots that illustrate the relationships between the running speed and the stride length and the relationships between the running speed and the cadence of multiple high cadence type runners. The horizontal axis represents the running speed [m/s], and the vertical axis represents the stride length [m] or cadence [spm].

FIG. 4A shows an example in which a fourth runner ran in a range of about 3.0 m/s (about 5 minutes 33 seconds per kilometer pace) to about 5.5 m/s (about 3 minutes 2 seconds per kilometer pace). In this case, the stride length plotted with "×" marks increases greatly by a range of about 0.65 m, from about 0.95 m to about 1.6 m, in proportion to the increase in speed. The slope of a stride length change 106, which is a regression line indicating the change in stride length with respect to the change in running speed, is relatively large. Meanwhile, the cadence plotted with "·" marks also increases by a wide range from 177 to 203 as the speed increases. A cadence change 107, which is a regression line indicating the change in pace with respect to the change in running speed, is also large.

FIG. 4B shows an example in which a fifth runner ran in a range of about 4.1 m/s (about 4 minutes 39 seconds per kilometer pace) to about 5.3 m/s (about 3 minutes 9 seconds per kilometer pace). In this case, the stride length plotted with "×" marks increases greatly by a range of about 0.3 m, from about 1.3 m to about 1.6 m, in proportion to the increase in speed. The slope of a stride length change 108, which is a regression line indicating the change in stride length with respect to the change in running speed, is relatively large. Meanwhile, the cadence plotted with "·" marks also increases by a wide range from 184 to 194 as the speed increases. A cadence change 109, which is a regression line indicating the change in pace with respect to the change in running speed, is also large.

As described above, it can be seen that long stride type runners tend to increase the running speed by greatly increasing the stride length rather than by greatly increasing the cadence. On the other hand, high cadence type runners tend to increase the running speed by greatly increasing the cadence while also increasing the stride length to some extent. The running style analysis system of the present embodiment judges whether the running style of the subject is the long stride type or the high cadence type, using an analysis method that focuses on which of the above two contrasting approaches the subject uses to increase his or her running speed.

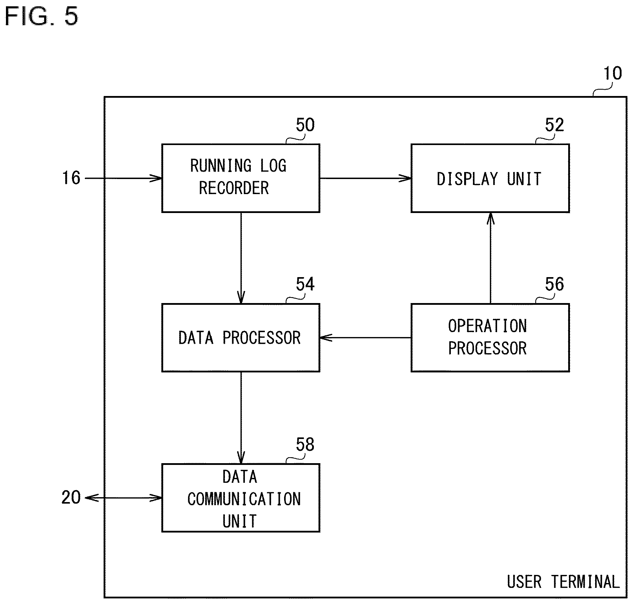

FIG. 5 is a functional block diagram that shows a basic configuration of the user terminal. FIG. 5 is a block diagram featuring the functions, and these functional blocks may be implemented in a variety of forms by hardware, software, or a combination thereof. The user terminal 10 may be, for example, an information terminal, such as a smartphone or a tablet, or a device such as a personal computer, in terms of hardware. The user terminal 10 includes at least each function of a running log recorder 50, a display unit 52, a data processor 54, an operation processor 56, and a data communication unit 58. As a modification, each function of the user terminal 10 shown in FIG. 5 may be built into a wearable device 16 so as to be implemented as an integrated device.

The running log recorder 50 acquires various detection data from a wearable device 16 via a communication module such as short-range wireless communication and records the data as a running log. The detection data acquired from a wearable device 16 include position information received from a satellite positioning system, such as the GPS (Global Positioning System), information indicating the acquisition date and time thereof, and information on the cadence per unit time (e.g., the number of steps per minute). Based on the detection data acquired from a wearable device 16, the running log recorder 50 records, as a running log, information such as the running time, running distance, running speed for each predetermined distance or each predetermined time, and cadence per unit time, in a predetermined storage area.

The operation processor 56 accepts operation input for an instruction from the user. Based on the user's instruction via the operation processor 56, the display unit 52 displays, on a screen, a running log recorded by the running log recorder 50. Also, based on the user's instruction via the operation processor 56, the data processor 54 extracts data of cadence at multiple running speeds from the running logs recorded by the running log recorder 50 and transmits the extracted data as the subject's running logs to the running style analysis server 20 via the data communication unit 58. For example, the data processor 54 extracts data of cadence at running speeds of 4.17 m/s (4 min/km pace) or higher from all the running logs and transmits the extracted data as the subject's running logs to the running style analysis server 20. The data of lower than 4.17 m/s is cut and not used because, during running at relatively high speeds, the relationship between the running speed and the cadence is more stable, and a clear trend appears. In a modification, the data processor 54 may transmit all the running logs to the running style analysis server 20 via the data communication unit 58, and necessary data may be extracted on the running style analysis server 20 side.

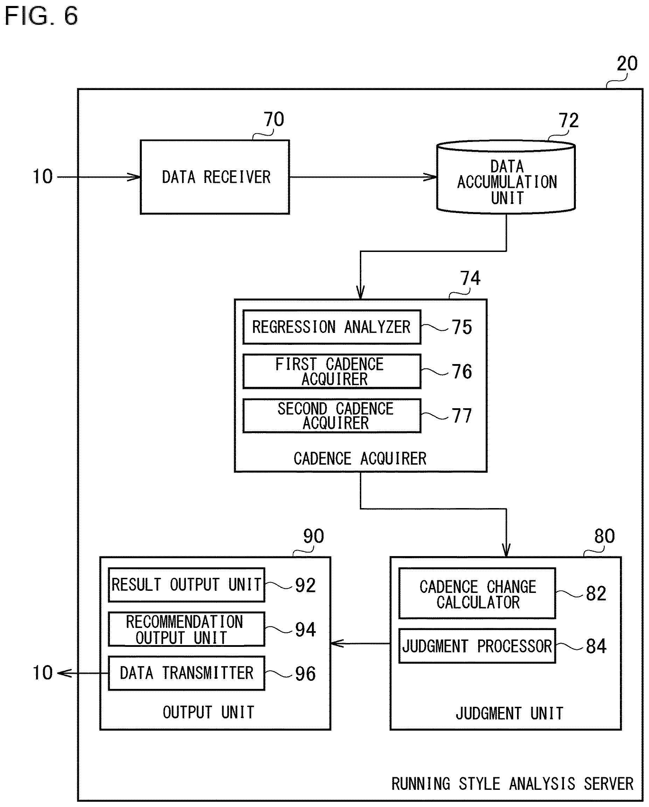

FIG. 6 is a functional block diagram that shows a basic configuration of the running style analysis server. FIG. 6 is a block diagram featuring the functions, and these functional blocks may be implemented in a variety of forms by hardware, software, or a combination thereof. The running style analysis server 20 may be, for example, a server computer in terms of hardware. The running style analysis server 20 includes at least each function of a data receiver 70, a data accumulation unit 72, a cadence acquirer 74, a judgment unit 80, and an output unit 90.

The data receiver 70 receives, from the user terminal 10, running speed data and cadence data included in a running log of the subject and stores those data in the data accumulation unit 72. In the present embodiment, the data receiver 70 receives, as a running log, the data of cadence at a running speed of 4.17 m/s or higher extracted by the data processor 54 of the user terminal 10. In a modification, the data receiver 70 may receive all the running logs of the subject and store the running logs in the data accumulation unit 72. With regard to the subject's running, the cadence acquirer 74 acquires the data of cadence at multiple running speeds from the subject's running logs stored in the data accumulation unit 72. The data of cadence acquired by the cadence acquirer 74 is, for example, data of cadence at a running speed of 4.17 m/s or higher. The cadence acquirer 74 includes a regression analyzer 75, a first cadence acquirer 76, and a second cadence acquirer 77.

The regression analyzer 75 obtains a regression equation by regression analysis using the cadence as the objective variable and the running speed as the explanatory variable, based on the running logs stored in the data accumulation unit 72. The regression analyzer 75 performs regression analysis on at least two data points as data indicating the relationship between the running speed and the cadence. The more data to be analyzed, the fewer the errors in the regression equation and the higher the accuracy, so that it is desirable to analyze three or more data points.

Based on the regression equation obtained by the regression analyzer 75, the first cadence acquirer 76 acquires the cadence at a first running speed, which is a predetermined running speed of 4.17 m/s (4 min/km pace) or higher. Also, based on the regression equation obtained by the regression analyzer 75, the second cadence acquirer 77 acquires the cadence at a second running speed, which is a running speed 1 m/s higher than the first running speed. Although the second running speed is set to a running speed 1 m/s higher than the first running speed in the present embodiment, the speed difference therebetween is not limited to 1 m/s. The first running speed and the second running speed may be set with a larger difference therebetween or may be set with a difference of less than 1 m/s as long as a certain difference is ensured.

The judgment unit 80 judges which of multiple running style types, including the long stride type and the high cadence type, the subject's running falls under. The judgment unit 80 includes a cadence change calculator 82 and a judgment processor 84. The cadence change calculator 82 calculates the difference between a first cadence and a second cadence with respect to the difference between the first running speed and the second running speed.

Based on the difference between the first cadence and the second cadence with respect to the difference between the first running speed and the second running speed, the judgment processor 84 judges which of multiple running style types, including the long stride type and the high cadence type, the subject's running falls under. First, when the cadence at an arbitrary running speed is greater than or equal to a predetermined judgment criterion value, the judgment processor 84 judges that the running falls under the high cadence type, regardless of the magnitude of the cadence change with respect to the running speed change. More specifically, with the running speed as x, the variable value obtained from the linear equation "9.6x + 145 spm" is used as the judgment criterion value. With the cadence as y, when y ≥ 9.6x + 145 spm, the judgment processor 84 judges that the running falls under the high cadence type. As a modification, a fixed value obtained in advance through an experiment or analysis, such as "190 spm", may be used as the judgment criterion value. In this case, the fixed value may be adopted only when the cadence is measured at a running speed of 5.56 m/s (3 min/km pace) or lower, for example. In general, as the running speed increases, even a long stride type runner may considerably increase the cadence to be comparable to a high cadence type runner. Therefore, rather than using a fixed value as the judgment criterion, it is desirable to use a variable value based on a linear equation proportional to the running speed as the judgment criterion in order to maintain the judgment accuracy. However, when the running speed (pace) is limited to about 3 min/km or less, it is possible to judge with high accuracy even with a fixed value as the judgment criterion.

When the cadence y is less than the judgment criterion value (y < 9.6x + 145 spm), if the difference (increase) between the first cadence and the second cadence before and after the running speed increases by 1 m/s is within a first range (e.g., 16 times or more), it will be judged as the high cadence type; if the difference (increase) is within a second range (less than 10 times), which is lower than the first range, it will be judged as the long stride type. If the difference between the first cadence and the second cadence is within a third range (10 times or more and less than 16 times), which is lower than the first reference range and higher than the second reference range, it will be judged as an intermediate type. In the case of the intermediate type, when the difference between the first cadence and the second cadence is 10 times or more and less than 13 times, it is judged as "rather on the long stride type side" or "the intermediate type close to the long stride type"; when the difference between the first cadence and the second cadence is 13 times or more and less than 16 times, it is judged as "rather on the high cadence type side" or "the intermediate type close to the high cadence type". In the case of the "intermediate type", although it is considered that wearing either shoes for the high cadence type or shoes for the long stride type will not have a significant effect on running, which one is more likely to improve performance somewhat is judged here.

The output unit 90 includes a result output unit 92, a recommendation output unit 94, and a data transmitter 96. The result output unit 92 outputs the result of the judgment by the judgment unit 80 to the user terminal 10 via the data transmitter 96. More specifically, the result output unit 92 transmits to the user terminal 10 the judgment result as to whether the subject's running style corresponds to the high cadence type, long stride type, or intermediate type so as to display the judgment result on the screen of the user terminal 10.

Based on the result of the judgment by the judgment unit 80, the recommendation output unit 94 selects shoes suitable for the subject's running style from among multiple options. The recommendation output unit 94 generates information recommending at least one of the multiple shoe candidates and outputs the information to the user terminal 10 via the data transmitter 96. When the subject is judged as the long stride type, the recommendation output unit 94 selects shoes suitable for long stride type runners. When the subject is judged as the high cadence type, the recommendation output unit 94 selects shoes suitable for high cadence type runners. Running shoes for the long stride type have better sole repulsion than those for the high cadence type, for example. When it is judged as the intermediate type and also as "rather on the long stride type side", recommendation information is generated that recommends both the shoes for the long stride type and the shoes for the high cadence type but recommends the shoes for the long stride type more strongly. Also, when it is judged as the intermediate type and also as "rather on the high cadence type side", recommendation information is generated that recommends both the shoes for the long stride type and the shoes for the high cadence type but recommends the shoes for the high cadence type more strongly.

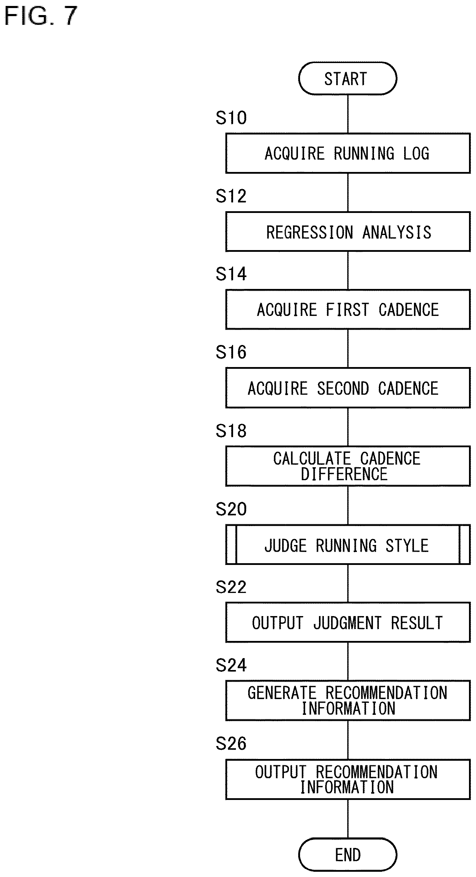

FIG. 7 is a flowchart that shows basic processing performed in the running style analysis server. The data receiver 70 acquires a running log of the subject (S10). The regression analyzer 75 performs regression analysis on the running log of the subject (S12). The first cadence acquirer 76 acquires the first cadence at the first running speed based on a regression equation (S14). The second cadence acquirer 77 acquires the second cadence at the second running speed, which is 1 m/s higher than the first running speed, based on the regression equation (S16). The cadence change calculator 82 calculates the magnitude of the cadence change (i.e., the increase in cadence) with respect to the running speed difference of 1 m/s between the first running speed and the second running speed (S18). The judgment processor 84 judges the running style based on the magnitude of the cadence or the magnitude of the cadence change (S20). The result output unit 92 outputs the judgment result from the judgment processor 84 to the user terminal 10 (S22). The recommendation output unit 94 determines shoes to recommend based on the judgment result from the judgment processor 84 and generates recommendation information (S24), and the recommendation output unit 94 outputs the recommendation information to the user terminal 10 (S26).

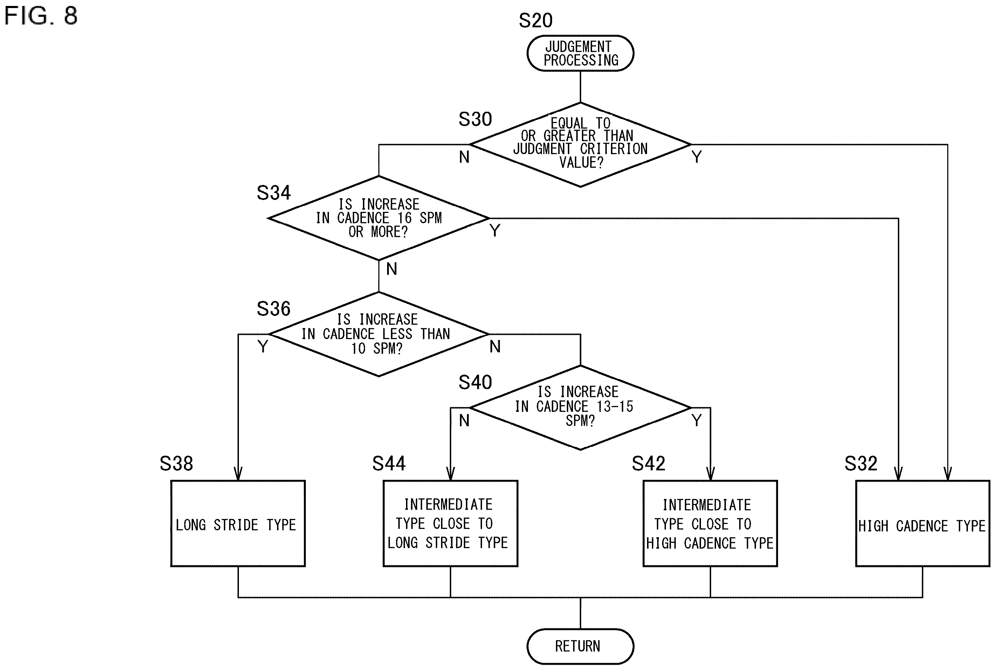

FIG. 8 is a flowchart that shows the details of the judgement processing at S20 in FIG. 7. With the running speed as x and the cadence as y, when the cadence y of the subject is greater than or equal to a judgment criterion value (9.6x + 145 spm) (Y at S30), the judgment processor 84 judges that the subject's running style falls under the high cadence type (S32). Even when the cadence y of the subject is less than the judgment criterion value (N at S30), if the increase in cadence with respect to the running speed difference of 1 m/s is within the first range (+16 spm or more) (Y at S34), the subject's running style is judged as the high cadence type (S32). At S34, when the increase in cadence with respect to the running speed difference of 1 m/s is less than +16 spm (N at S34) and when the increase in cadence is within the second range (less than +10 spm) (Y at S36), the subject's running style is judged as the long stride type (S38). When the increase in cadence is +10 spm or more (N at S36) and when it is +13 spm or more and +15 spm or less (less than 16 spm) (Y at S40), the subject's running style is judged as the intermediate type close to the high cadence type (S42). When the increase in cadence is not +13 spm or more and +15 spm or less (less than 16 spm), i.e., when the increase in cadence is +10 spm or more and less than +13 spm (N at S40), the subject's running style is judged as the intermediate type close to the long stride type (S44).

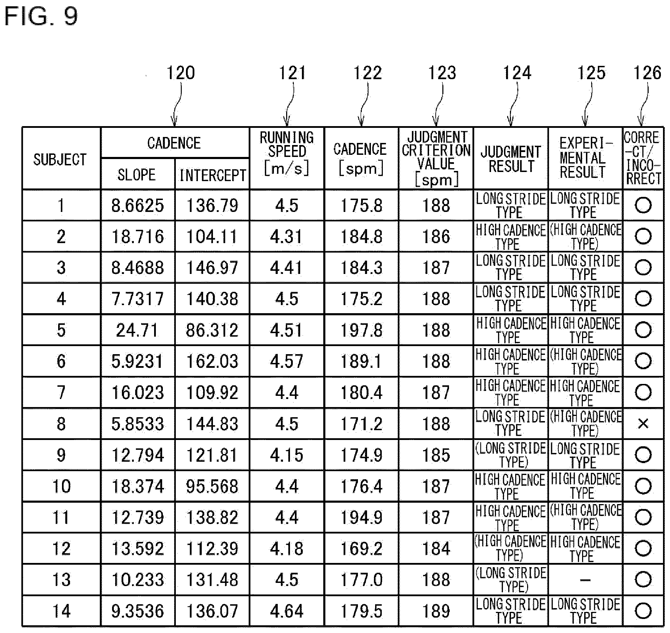

FIG. 9 is a diagram in which results of running style judgment based on the cadence are compared with experimental results, with regard to 14 subjects. A running log has been obtained for each of the 14 subjects numbered "1" to "14", and the results of regression analysis with the cadence as the objective variable and the running speed as the explanatory variable are shown in a first column 120. The first column 120 shows the slope and intercept of each regression equation obtained by regression analysis. Based on the regression equations, the cadence at each of the first running speed and the second running speed and the difference therebetween of each subject can be obtained. A running speed (race pace) as an actual measured value included in a running log is shown in a second column 121, and the cadence as an actual measured value at the running speed is shown in a third column 122. The judgment criterion value (9.6x + 145 spm), where x is the running speed shown in the second column 121, is shown in a fourth column 123. When the cadence shown in the third column 122 is greater than or equal to the judgment criterion value shown in the fourth column 123 (Y at S30 in FIG. 8), the subject's running style is immediately judged as the high cadence type (S32 in FIG. 8). A fifth column 124 shows the judgment result as to the subject's running style. For example, with regard to each of the subjects "5", "6", and "11", the cadence shown in the third column 122 is greater than or equal to the judgment criterion value shown in the fourth column 123, so that the subjects are immediately judged as the high cadence type, and the judgment result of "high cadence type" is shown in the fifth column 124 for each of the subjects. With regard to each of the other subjects "1"-"4", "7"-"10", and "12"-"14", the increase in cadence with respect to the running speed difference of 1 m/s is obtained based on the regression equation shown in the first column 120, and the result judged based on the judgement processing shown in FIG. 8 is shown in the fifth column 124. Among the judgement results shown in the fifth column 124, the "(high cadence type)", indicated with parentheses, means the judgement result of the "intermediate type close to the high cadence type", and the "(long stride type)", indicated with parentheses, means the judgement result of the "intermediate type close to the long stride type".

A sixth column 125 shows an experimental result of a running experiment performed by each subject actually wearing each of shoes for the high cadence type and shoes for the long stride type, indicating which running style type of shoes the subject could run faster with. The judgement result shown in the fifth column 124 and the experimental result shown in the sixth column 125 are compared, and, when the results match each other, "o" is shown as a correct/incorrect result in a seventh column 126; when the results do not match each other, "×" is shown as the correct/incorrect result in the seventh column 126. With regard to the experimental result of the subject "13", since no difference has been observed between the shoes for the high cadence type and the shoes for the long stride type, "-" is shown to indicate that either the high cadence type shoes or the long stride type shoes may be used. According to the above, except for one subject "8", the experimental results of 13 out of 14 subjects match with their judgment results, which confirms that the judgement processing shown in FIG. 8 enables accurate judgement.

The present invention has been described with reference to an embodiment. The embodiment is intended to be illustrative only, and it will be obvious to those skilled in the art that various modifications to a combination of constituting elements or processes could be developed. In the following, modifications will be described.

The abovementioned embodiment describes an example in which a running log is acquired by a wearable device 16, and running style analysis is performed based on the running log. In a modification, the user may enter numerical values of cadence for at least two running speeds, in the form of text entry in a predetermined field on a web browser, to send the information to the running style analysis server 20, so as to allow the running style analysis server 20 to perform running style analysis.

The abovementioned embodiment describes an example in which running style analysis is performed with the running style analysis system 30 including the user terminal 10 and the running style analysis server 20. In a modification, each function for the running style analysis may be implemented on a device, such as a smartphone, tablet, or personal computer, directly operated by the user, rather than on the running style analysis server 20.

## Claims

1. A running style analysis device (20), comprising:
a first cadence acquirer (76) configured to acquire, with regard to running of a subject, a cadence at a first running speed as a first cadence;
a second cadence acquirer (77 configured to acquire, with regard to running of the subject, a cadence at a second running speed that is different from the first running speed, as a second cadence;
a judgment unit (80) configured to make a judgment as to which of a plurality of running style types, which include a long stride type and a high cadence type, running of the subject falls under, based on the difference between the first cadence and the second cadence with respect to the difference between the first running speed and the second running speed; and
a result output unit (92) configured to output a result of the judgment.

2. The running style analysis device (20) according to claim 1, further configured so that, when at least one of the first cadence or the second cadence is greater than or equal to a judgment criterion value obtained based on a predetermined criterion, the judgment unit (80) judges that running of the subject falls under the high cadence type, regardless of the difference in cadence.

3. The running style analysis device (20) according to claim 1 or 2, wherein the judgment unit (80) is further configured to judge that running of the subject falls under the high cadence type when the difference in cadence is within a predetermined first reference range and that running of the subject falls under the long stride type when the difference in cadence is within a predetermined second reference range, which is lower than the first reference range.

4. The running style analysis device (20) according to claim 1 or 2, wherein the judgment unit (80) is further configured to judge that running of the subject falls under the high cadence type when the difference in cadence is within a predetermined first reference range, judges that running of the subject falls under the long stride type when the difference in cadence is within a predetermined second reference range, which is lower than the first reference range, and that running of the subject falls under an intermediate type when the difference in cadence is within a predetermined third reference range, which is lower than the first reference range and higher than the second reference range.

5. The running style analysis device (20) according to any one of claims 1 through 4, further comprising a recommendation output unit (94) configured to output, based on a result of the judgment, information recommending at least one of a plurality of shoes including a shoe suitable for a high cadence type runner and a shoe suitable for a long stride type runner.

6. A computer-implemented running style analysis method for execution on a device as defined in claim 1 comprising:
acquiring, with regard to running of a subject, a cadence at a first running speed as a first cadence;
acquiring, with regard to running of the subject, a cadence at a second running speed that is different from the first running speed, as a second cadence;
making judgment as to which of a plurality of running style types, which include a long stride type and a high cadence type, running of the subject falls under, based on the difference between the first cadence and the second cadence with respect to the difference between the first running speed and the second running speed; and
outputting a result of the judgment.

7. A running style analysis program causing a computer on a device as defined in claim 1 to implement:
acquiring, with regard to running of a subject, a cadence at a first running speed as a first cadence;
acquiring, with regard to running of the subject, a cadence at a second running speed that is different from the first running speed, as a second cadence;
making judgment as to which of a plurality of running style types, which include a long stride type and a high cadence type, running of the subject falls under, based on the difference between the first cadence and the second cadence with respect to the difference between the first running speed and the second running speed; and
outputting a result of the judgment.

## Patentansprüche

1. Laufstil-Analysevorrichtung (20), die Folgendes umfasst:
einen ersten Schrittfrequenzerfasser (76), der dafür konfiguriert ist, in Bezug auf den Lauf einer Testperson eine Schrittfrequenz bei einer ersten Laufgeschwindigkeit als eine erste Schrittfrequenz zu erfassen,
einen zweiten Schrittfrequenzerfasser (77), der dafür konfiguriert ist, in Bezug auf den Lauf der Testperson eine Schrittfrequenz bei einer zweiten Laufgeschwindigkeit, die sich von der ersten Laufgeschwindigkeit unterscheidet, als eine zweite Schrittfrequenz zu erfassen,
eine Beurteilungseinheit (80), die dafür konfiguriert ist, eine Beurteilung darüber zu treffen, unter welchen von einer Vielzahl von Laufstiltypen, die einen Typ mit langen Schritten und einen Typ mit hoher Schrittfrequenz einschließt, der Lauf der Testperson fällt, und zwar auf der Grundlage der Differenz zwischen der ersten Schrittfrequenz und der zweiten Schrittfrequenz in Bezug auf die Differenz zwischen der ersten Laufgeschwindigkeit und der zweiten Laufgeschwindigkeit, und
eine Ergebnisausgabeeinheit (92), die dafür konfiguriert ist, ein Ergebnis der Beurteilung auszugeben.

2. Laufstil-Analysevorrichtung (20) nach Anspruch 1, die ferner so konfiguriert ist, dass, wenn mindestens eine von der ersten Schrittfrequenz oder der zweiten Schrittfrequenz größer als oder so groß wie ein auf der Grundlage eines vorbestimmten Kriteriums erhaltener Beurteilungskriteriumswert ist, die Beurteilungseinheit (80) urteilt, dass der Lauf der Testperson unter den Typ mit hoher Schrittfrequenz fällt, und zwar ungeachtet der Differenz bei der Schrittfrequenz.

3. Laufstil-Analysevorrichtung (20) nach Anspruch 1 oder 2, wobei die Beurteilungseinheit (80) ferner dafür konfiguriert ist, zu urteilen, dass der Lauf der Testperson unter den Typ mit hoher Schrittfrequenz fällt, wenn die Differenz bei der Schrittfrequenz innerhalb eines vorbestimmten ersten Referenzbereichs liegt, und dass der Lauf der Testperson unter den Typ mit langen Schritten fällt, wenn die Differenz bei der Schrittfrequenz innerhalb eines zweiten Referenzbereichs liegt, der niedriger als der erste Referenzbereich ist.

4. Laufstil-Analysevorrichtung (20) nach Anspruch 1 oder 2, wobei die Beurteilungseinheit (80) ferner dafür konfiguriert ist, zu urteilen, dass der Lauf der Testperson unter den Typ mit hoher Schrittfrequenz fällt, wenn die Differenz bei der Schrittfrequenz innerhalb eines vorbestimmten ersten Referenzbereichs liegt, zu urteilen, dass der Lauf der Testperson unter den Typ mit langen Schritten fällt, wenn die Differenz bei der Schrittfrequenz innerhalb eines zweiten Referenzbereichs liegt, der niedriger als der erste Referenzbereich ist, und zu urteilen, dass der Lauf der Testperson unter einen Zwischentyp fällt, wenn die Differenz bei der Schrittfrequenz innerhalb eines dritten Referenzbereichs liegt, der niedriger als der erste Referenzbereich und höher als der zweite Referenzbereich ist.

5. Laufstil-Analysevorrichtung (20) nach einem der Ansprüche 1 bis 4, die ferner eine Empfehlungsausgabeeinheit (94) umfasst, die dafür konfiguriert ist, auf der Grundlage eines Ergebnisses der Beurteilung Informationen auszugeben, die mindestens einen von einer Vielzahl von Schuhen, die einen für einen Läufer des Typs mit hoher Schrittfrequenz geeigneten Schuh und einen für einen Läufer des Typs mit langen Schritten geeigneten Schuh einschließt, empfehlen.

6. Rechnerimplementiertes Laufstil-Analyseverfahren zur Ausführung auf einer Vorrichtung nach Anspruch 1, das Folgendes umfasst:
Erfassen, in Bezug auf den Lauf einer Testperson, einer Schrittfrequenz bei einer ersten Laufgeschwindigkeit als eine erste Schrittfrequenz,
Erfassen, in Bezug auf den Lauf der Testperson, einer Schrittfrequenz bei einer zweiten Laufgeschwindigkeit, die sich von der ersten Laufgeschwindigkeit unterscheidet, als eine zweite Schrittfrequenz,
Treffen einer Beurteilung darüber, unter welchen von einer Vielzahl von Laufstiltypen, die einen Typ mit langen Schritten und einen Typ mit hoher Schrittfrequenz einschließt, der Lauf der Testperson fällt, und zwar auf der Grundlage der Differenz zwischen der ersten Schrittfrequenz und der zweiten Schrittfrequenz in Bezug auf die Differenz zwischen der ersten Laufgeschwindigkeit und der zweiten Laufgeschwindigkeit, und
Ausgeben eines Ergebnisses der Beurteilung.

7. Laufstil-Analyseprogramm, das veranlasst, dass ein Rechner auf einer Vorrichtung nach Anspruch 1 Folgendes umsetzt:
Erfassen, in Bezug auf den Lauf einer Testperson, einer Schrittfrequenz bei einer ersten Laufgeschwindigkeit als eine erste Schrittfrequenz,
Erfassen, in Bezug auf den Lauf der Testperson, einer Schrittfrequenz bei einer zweiten Laufgeschwindigkeit, die sich von der ersten Laufgeschwindigkeit unterscheidet, als eine zweite Schrittfrequenz,
Treffen einer Beurteilung darüber, unter welchen von einer Vielzahl von Laufstiltypen, die einen Typ mit langen Schritten und einen Typ mit hoher Schrittfrequenz einschließt, der Lauf der Testperson fällt, auf der Grundlage der Differenz zwischen der ersten Schrittfrequenz und der zweiten Schrittfrequenz in Bezug auf die Differenz zwischen der ersten Laufgeschwindigkeit und der zweiten Laufgeschwindigkeit, und
Ausgeben eines Ergebnisses der Beurteilung.

## Revendications

1. Dispositif d'analyse de style de course (20), comprenant :
un premier acquéreur de cadence (76) configuré pour acquérir, en ce qui concerne la course d'un sujet, une cadence à une première vitesse de course comme première cadence ;
un deuxième acquéreur de cadence (77) configuré pour acquérir, en ce qui concerne la course du sujet, une cadence à une deuxième vitesse de course, différente de la première vitesse de course, comme deuxième cadence ;
une unité de jugement (80) configurée pour établir un jugement visant à savoir de quel type, parmi une pluralité de types de style de course dont un type à longues foulées et un type à cadence élevée, relève la course du sujet, compte tenu de la différence entre la première cadence et la deuxième cadence par rapport à la différence entre la première vitesse de course et la deuxième vitesse de course ; et
une unité de production de résultat (92) configurée pour produire un résultat du jugement.

2. Dispositif d'analyse de style de course (20) selon la revendication 1, configuré en outre de façon que, lorsqu'au moins une cadence parmi la première cadence et la deuxième cadence est supérieure ou égale à une valeur de critère de jugement obtenue compte tenu d'un critère prédéterminé, l'unité de jugement (80) juge que la course du sujet relève du type à cadence élevée, indépendamment de la différence de cadence.

3. Dispositif d'analyse de style de course (20) selon la revendication 1 ou 2, dans lequel l'unité de jugement (80) est en outre configurée pour juger que la course du sujet relève du type à cadence élevée lorsque la différence de cadence est comprise dans une première plage de référence prédéterminée, et que la course du sujet relève du type à longues foulées lorsque la différence de cadence est comprise dans une deuxième plage de référence prédéterminée inférieure à la première plage de référence.

4. Dispositif d'analyse de style de course (20) selon la revendication 1 ou 2, dans lequel l'unité de jugement (80) est en outre configurée pour juger que la course du sujet relève du type à cadence élevée lorsque la différence de cadence est comprise dans une première plage de référence prédéterminée, et pour juger que la course du sujet relève du type à longues foulées lorsque la différence de cadence est comprise dans une deuxième plage de référence prédéterminée inférieure à la première plage de référence, et que la course du sujet relève d'un type intermédiaire lorsque la différence de cadence est comprise dans une troisième plage de référence prédéterminée qui est inférieure à la première plage de référence et supérieure à la deuxième plage de référence.

5. Dispositif d'analyse de style de course (20) selon l'une quelconque des revendications 1 à 4, comprenant en outre une unité de production de recommandation (94) configurée pour produire, compte tenu d'un résultat du jugement, des informations recommandant au moins une chaussure parmi une pluralité de chaussures incluant une chaussure adaptée à un coureur du type à cadence élevée et une chaussure adaptée à un coureur du type à longues foulées.

6. Procédé d'analyse de style de course mis en œuvre par ordinateur et destiné à être exécuté sur un dispositif défini dans la revendication 1, comprenant :
l'acquisition, en ce qui concerne la course d'un sujet, d'une cadence à une première vitesse de course comme première cadence ;
l'acquisition, en ce qui concerne la course du sujet, d'une cadence à une deuxième vitesse de course, différente de la première vitesse de course, comme deuxième cadence ;
l'établissement d'un jugement visant à savoir de quel type, parmi une pluralité de types de style de course dont un type à longues foulées et un type à cadence élevée, relève la course du sujet, compte tenu de la différence entre la première cadence et la deuxième cadence par rapport à la différence entre la première vitesse de course et la deuxième vitesse de course ; et
la production d'un résultat du jugement.

7. Programme d'analyse de style de course amenant un ordinateur, sur un dispositif défini dans la revendication 1, à mettre en œuvre :
l'acquisition, en ce qui concerne la course d'un sujet, d'une cadence à une première vitesse de course comme première cadence ;
l'acquisition, en ce qui concerne la course du sujet, d'une cadence à une deuxième vitesse de course, différente de la première vitesse de course, comme deuxième cadence ;
l'établissement d'un jugement visant à savoir de quel type, parmi une pluralité de types de style de course dont un type à longues foulées et un type à cadence élevée, relève la course du sujet, compte tenu de la différence entre la première cadence et la deuxième cadence par rapport à la différence entre la première vitesse de course et la deuxième vitesse de course ; et
la production d'un résultat du jugement.
